(19) Europäisches Patentamt

European Patent Office

Office européen des brevets

(11) Publication number: **0 259 927 B1**

(12) **EUROPEAN PATENT SPECIFICATION**

(45) Date of publication of patent specification: **06.05.92**  (51) Int. Cl.⁵: **C07C 1/00**

(21) Application number: **87201683.7**

(22) Date of filing: **07.09.87**

(54) **Process to produce a high methane content gas mixture from coal.**

(30) Priority: **10.09.86 IT 2166186**

(43) Date of publication of application:
**16.03.88 Bulletin 88/11**

(45) Publication of the grant of the patent:
**06.05.92 Bulletin 92/19**

(84) Designated Contracting States:
**BE DE ES FR GB NL**

(56) References cited:
**EP-A- 0 067 580**
**GB-A- 2 055 892**
**US-A- 3 689 240**

(73) Proprietor: **ENIRICERCHE S.p.A.**
**Corso Venezia 16**
**I-20121 Milan(IT)**

Proprietor: **SNAM S.p.A.**
**Corso Venezia 16**
**I-20121 Milan(IT)**

(72) Inventor: **Bruno, Giacomo**
**Via Dogali 30**
**I-16038 Santa Margherita Ligure Genova(IT)**
Inventor: **Carvani, Luigi**
**Via 4 Novembre 110**
**I-29100 Piacenza(IT)**
Inventor: **Passoni, Giovanni**
**Via Bordolano 2B**
**I-20097 San Donato Milanese Milan(IT)**

(74) Representative: **Henke, Erwin et al**
**Ing.Barzanò & Zanardo Milano S.p.A. Via Borgonuovo, 10**
**I-20121 Milano(IT)**

## Description

The present invention relates to a process to obtain from coal a gas mixture with a high methane content.

More particularly, the present invention relates to a process to obtain from coal a high methane content gas mixture, wherein the gasification and methanation reactions take place in one single, descending fixed-bed reactor.

The high methane content gas mixtures which are produced by means of said process are normally denominated "SNG" (substitute natural gas) and can either replace the natural gas, or be mixed with it.

Process have been long known, which start from coal, to produce the above-mentioned mixtures.

At Great Plains (U.S.A.), e.g., a process has been accomplished, which essentially comprises the following steps:

- grinding of coal down to a size comprised within the range of from 6 mm to 5 cm;
- fixed-bed gasification by means of oxygen and steam under high pressures (of approximately 89,4 bar (30 abs.atm) and at temperatures comprised within the range of from 500°C to 1200°C;
- condensation of tars, unreacted water and condensible compounds formed;
- shift of a portion of the produced gas, to adjust to a value of approximately 3 the $H_2/CO$ volume ratio;
- removal of the acidic gases;
- methanation of the product at a temperature of approximately 400°C.

The process shows some drawbacks, mainly caused by the need of feeding to the gasification step large amounts of essentially pure oxygen (in amounts larger than 50% by weight relatively to the dry, ash-free coal), in as mush as the use of air would involve the need for nitrogen to be removed after the methanation step.

The need to have available essentially pure oxygen involves the presence of an air fractionation facility, which causes high fixed costs and high operation costs, due to the large amounts of oxygen to be supplied.

Such process is generally very complex, precisely due to the different steps which constitute it.

EP-A-0 067 580 on the contrary, designed a process wherein the reactions of gasification and methanation take place in one single step, which essentially comprises the following steps:

- grinding of coal to a particle size not larger than 3 mm;
- impregnation of the ground coal with water and a catalyst;
- gasification and methanation by means of steam on a catalytic fluidized bed at a temperature of approximately 700°C;
- removal of the acidic gases from the gas mixture formed;
- cryogenic separation, to decrease in the gas mixture the contents of $H_2$ and CO, which are recycled to the gasification and methanation step;
- lixiviation with water of the gasification residue, to partially recover the catalyst (from 70% to 90% per each cycle).

The most serious defects of the process of EP-A-0 067 580 are that it requires an expensive cryogenic purification and the recycling of hydrogen and carbon monoxide, plus the necessity of overheating to maintain the desired temperature, whereby the gassification efficiency is reduced. Moreover, the adoption of a bulky fluidized bed originates problems and limits the residence time of the gas in the reactor.

US-A-3 689 240 generally discloses the use of alkali metal catalysts in methanizing a carbonaceous material and, in addition to using steam only, without any added air or oxygen, considers the fixed bed technique, the moving bed technique and the fluidized bed technique as virtually equivalent.

Applicants have surprisingly found a simplified process in which methanization and gassification are a single step, so that the installation first costs are drastically reduced.

The invention, therefore, provides a process for preparing a high-methane-content gas from coal by conversion with steam and oxygen or an oxygen-containing gas, in the presence of an alkali metal- or an alkaline earth metal compound as the catalyst, characterized by comprising the steps of:

- grinding the coal to a particle size not greater than 5 mm;
- impregnating the ground coal with an aqueous solution of the catalyst, in which the alkali metal- or the alkaline earth metal ions are present in an amount of from 4% to 20% by weight relative to the dry, ash-free coal, optionally drying the impregnated coal, optionally pelletizing the catalyst-impregnated coal, to a pellet size of from 3 mm to 5 cm;

EP 0 259 927 B1

- methanizing the catalyst-impregnated coal by gassifying a catalyst-impregnated coal bed descending in a reactor in countercurrent relationship with the steam and oxygen or air stream, the air content being not greater than 35% by weight of the dry, ash-free coal, or the oxygen content being from 1% to 25% by weight of the dry, ash-free coal, under a pressure higher than 24,5165 bar (25 abs.atm.) and at a temperature of from 400°C to 850°C;
- stripping the unreacted steam and the condensibles from the as-produced gas, and
- deacidifying the gas so produced.

By coal, also other carbonaceous materials are intended, such as char, charcoal, coke and lignite.

By pelletizing not only cylindrical tablets, but also other geometric solids can be obtained, such as prisms, parallelepipeds and pellets proper.

Methanization by gassification take place in a reactor in which the gases to be reacted enter through a bottom inlet at a high temperature, the temperature decreasing gradually towards the reator top where the coal in fed: such a temperature trend encourages gassification in the bottom section and methanization in the top section of the reactor.

The catalyst is preferably added to the ground coal by impregnating it with an aqueous solution of one or more alkali metal-, or alkaline earth metal compound, which can be partially recovered by means of the lixiviation with water of the gasification residue, and recycled to the addition step.

The alkali and/or alkali-earth compounds can be selected from the salts, hydroxides or oxides of alkali and alkali-earth metals, such as, e.g., $K_2CO_3$, KOH, NaOH, CaO.

The flowrate of steam used in the gasification and methanation step is preferably comprised within the range of from 100 to 200% by weight relatively to the feed rate of dry, ash-free coal.

The residence time inside the gasification and methanation step is preferably comprised within the range of from 0.5 to 30 hours, more. preferably of from 1 to 5 hours.

Sometimes, a further end step can be necessary to reduce the CO content inside the obtained mixture.

This latter possible step consists in an end finishing methanation carried out by operating at a temperature comprised within the range of from 350° to 500°C over such catalysts as, e.g., nickel.

The concentration of the various components in said mixtures depends, above all, on the type of coal used and on the use of air and/or oxygen to carry out the gasification.

As relates to the use of air instead on oxygen and vice-versa, the selection between the two operating modes is influenced by the required percentage of oxygen relatively to the dry, ash-free coal to carry out the gasification and the methanation.

It is preferable to select air when the required oxygen percentage is lower then 5% by weight.

The invention is now illustrated with the aid of the hereto attached figure, which shows a preferred practical embodiment thereof.

Coal (1), after being ground, is impregnated in (2) with an aqueous solution of one or more alkaline and/or alkaline-earth compounds (3), and is subsequently dried.

The impregnated and dried coal (4) is possibly pelletized in (5), to the desired size.

Coal (6) is then converted into methane (7) by means of steam (8) and air (9) and/or oxygen (10). In case of oxygen feed, an air fractionation facility (11) is used to remove nitrogen (12).

The product (13) obtained from gasification and methanation is separated in (14) from the unreacted steam and from the incondensible compounds formed (15); and in (16) from the acidic gases (17) contained in it, the high methane content gas mixture (18) (SNG) being finally obtained.

The gasification residue (19) is recovered in (20) by lixiviating it with water (21) and is sent to (2) to impregnate coal.

In as much as the recovery is not total, a catalyst portion must be integrated by means of the line (3).

In order to better illustrate the meaning of the present invention, two Examples are reported.

Example 1
- - - - -

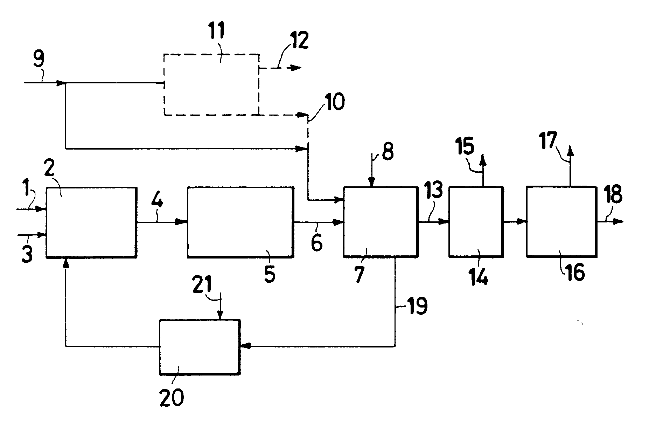

The test was carried out according to the diagram of the Figure, without the pelletizing step, and using essentially pure oxygen.

Charge:
- - - -

Type:         Char from Illinois 6, obtained by adding $K_2CO_3$ to coal and clarifying at 700°C for 60 minutes.
Particle size:  841$\mu$m-149$\mu$m (50-100 mesh.)
- - - - - - -

3

| Composition | C | 70,6% by weight |
| | H | 1,2% by weight |
| | O | 0,2% by weight (by difference) |
| | N | 1,5% by weight |
| | S | 3,0% by weight |
| | ashes | 23.5% by weight (comprising 11.5% of K ion) |

Gasification and Methanation Step Operating Conditions:

| Pressure | 35,30 bar (36 abs.atm) |
| $H_2O$ flowrate | 5,7 g/h |
| $O_2$ flowrate | 0,46 g/h |
| $O_2$ inlet temperature | 827 ° C |
| Steam inlet temperature | 827 ° C |

- Char was pre-heated to approximately 720 ° C, with the combustion reaction being started with pre-heated oxygen.
- Char temperature in the end portion of the reactor: 500 ° C.

A 1,5-metre high reactor with an inner diameter of 26 mm (adiabatic in the first half, partially thermally-insulated in the second half) was used.

Composition of the high methane content mixture obtained at 35,30 bar (36 abs.atm), excluding the nitrogen and sulphur containing gases:

| | After Cooling | | After Washing with NaOH Solution, % by volume |
| --- | --- | --- | --- |
| | mol/h.$10^3$ | g | |
| $H_2$ | 12,96 | 0,03 | 9,18 |
| CO | 4,32 | 0,12 | 3,06 |
| $CO_2$ | 129,70 | 5,7 | -- |
| $CH_4$ | 123,91 | 2,0 | 87,76 |

Example 2

The test was carried out according to the diagram of the Figure, without the pelletizing step, and using air.

Charge:

Type, Particle size, and Composition: same as of Example 1.

Gasification and Methanation Step Operating Conditions:

| Pressure | 98,066 bar (100 abs.atm) |
| $H_2O$ flowrate | 5,7 g/h |
| Air flowrate | 0,94 g/h |
| Air inlet temperature | 827 ° C |
| Steam inlet temperature | 827 ° C |

4

- Char was pre-heated to approximately 720°C, with the combustion reaction being started with pre-heated air.
- Char temperature in the end portion of the reactor: 500°C.

The same reactor as in Example 1 was used.

Composition of the high methane content mixture obtained at 98,066 bar (100 abs.atm), excluding the gases containing the nitrogen and sulphur initially present in coal:

| | After Cooling | | After Washing with NaOH Solution, % by volume |
|---|---|---|---|
| | mol/h.$10^3$ | weight, g | |
| $H_2$ | 8.25 | 0,02 | 5,19 |
| CO | 2.42 | 0,07 | 1,52 |
| $CO_2$ | 120.76 | 5,31 | -- |
| $CH_4$ | 123.29 | 1,97 | 77,49 |
| $N_2$ | 25.14 | 0,70 | 15,80 |

## Claims

1. Process for preparing a high-methane-content gas from coal by conversion with steam and oxygen or an oxygen-containing gas, in the presence of an alkali metal- or an alkaline earth metal compound as the catalyst, characterized by comprising the steps of:
   - grinding the coal to a particle size not greater than 5 mm;
   - impregnating the ground coal with an aqueous solution of the catalyst, in which the alkali metal- or the alkaline earth metal ions are present in an amount of from 4% to 20% by weight relative to the dry, ash-free coal, optionally drying the impregnated coal, optionally pelletizing the catalyst-impregnated coal, to a pellet size of from 3 mm to 5 cm;
   - methanizing the catalyst-impregnated coal by gassifying a catalyst-impregnated coal bed descending in a reactor in countercurrent relationship with the steam and oxygen or air stream, the air content being not greater than 35% by weight of the dry, ash-free coal, or the oxygen content being from 1% to 25% by weight of the dry, ash-free coal, under a pressure higher than 24,5165 bar (25 abs.atm.) and at a temperature of from 400°C to 850°C;
   - stripping the unreacted steam and the condensibles from the as-produced gas, and
   - deacidifying the gas so produced.

2. Process according to Claim 1, wherein the methanizing step is carried out at a temperature of from 450°C to 750°C.

3. Process according to Claim 1, wherein the pellet size of the catalyst-impregnated coal is of from 3 mm to 2 cm;

4. Process according to Claim 1, further comprising a supplemental methanizing step carried out at a temperature of from 350°C to 500°C.

5. Process according to claim 1, wherein the methanizing step is carried out with an amount of steam of from 100% to 200% by weigh relative to the dry, ash-free coal.

6. Process according to Claim 1, wherein the residence time of the coal to be methanized in the methanizing reactor is from 30 min to 30 hours.

7. Process according to Claim 7, wherein the residence time is from 1 hour to 5 hours.

8. Process accorsing to Claim 1, wherein the methanizing step takes place in a reactor in which the temperature increases from the reactor's top to the reactor's bottom.

**Revendications**

1.  Procédé de préparation d'un gaz à haute teneur en méthane à partir d'un charbon, par conversion avec de la vapeur d'eau et de l'oxygène ou un gaz contenant de l'oxygène, en présence d'un composé de métal alcalin ou de métal alcalino-terreux en tant que catalyseur, procédé caractérisé en ce qu'il comporte les étapes consistant à :
    -   broyer le charbon jusqu'à une dimension de particules non supérieure à 5 mm;
    -   imprégner le charbon broyé d'une solution aqueuse du catalyseur, dans laquelle les ions de métal alcalin ou de métal alcalino-terreux sont présents en une quantité de 4 % à 20 % en poids par rapport au charbon sec exempt de cendres, éventuellement sécher le charbon imprégné, éventuellement mettre le charbon imprégné de catalyseur sous forme de pastilles ayant une dimension de 3 mm à 5 cm;
    -   réaliser la méthanation du charbon imprégné de catalyseur, par gazéification d'un lit de charbon imprégné de catalyseur, descendant dans un réacteur à contre-courant du flux de vapeur d'eau et d'oxygène ou d'air, la teneur en air n'étant pas supérieure à 35 % en poids par rapport au charbon sec exempt de cendres ou la teneur en oxygène valant de 1 % à 25 % en poids par rapport au charbon sec exempt de cendres, sous une pression supérieure à 24,5165 bar (25 atm abs) et à une température de 400°C à 850°C;
    -   débarrasser le gaz produit brut de la vapeur d'eau n'ayant pas réagi et des matières condensables; et
    -   désacidifier le gaz ainsi produit.

2.  Procédé conforme à la revendication 1, dans lequel l'étape de méthanation est réalisée à une température de 450°C à 750°C.

3.  Procédé conforme à la revendication 1, dans lequel la taille des pastilles de charbon imprégné de catalyseur vaut de 3 mm à 2 cm.

4.  Procédé conforme à la revendication 1, comprenant en outre une étape supplémentaire de méthanation réalisée à une température de 350°C à 500°C.

5.  Procédé conforme à la revendication 1, dans lequel l'étape de méthanation est effectuée avec une quantité de vapeur d'eau valant de 100 % à 200 % en poids par rapport au charbon sec exempt de cendres.

6.  Procédé conforme à la revendication 1, dans lequel le temps de séjour du charbon à transformer en méthane dans le réacteur de méthanation est de 30 minutes à 30 heures.

7.  Procédé conforme à la revendication 6, dans lequel le temps de séjour est de 1 heure à 5 heures.

8.  Procédé conforme à la revendication 1, dans lequel l'étape de méthanation a lieu dans un réacteur dans lequel la température augmente depuis le sommet du réacteur jusqu'au fond du réacteur.

**Patentansprüche**

1.  Verfahren zur Herstellung eines Gases mit hohem Methangehalt aus Kohle durch Konversion mit Dampf und Sauerstoff oder einem sauerstoffhältigen Gas in Gegenwart einer Alkalimetall- oder einer Erdalkalimetallverbindung als Katalysator, durch gekennzeichnet, daß es die folgenden Stufen umfaßt:
    -   Mahlen der Kohle auf eine Teilchengröße von nicht über 5 mm;
    -   Imprägnieren der gemahlenen Kohle mit einer wäßrigen Lösung des Katalysators, worin die Alkalimetall- oder die Erdalkalimetallionen in einer Menge von 4 bis 20 Gew.-%, bezogen auf die trockene, aschefreie Kohle vorliegen, gegebenenfalls Trocknen der imprägnierten Kohle und gegebenenfalls Pelletisieren der mit dem Katalysator imprägnierten Kohle zu einer Pelletgröße von 3 mm bis 5 cm;
    -   Mathanisieren der mit dem Katalysator imprägnierten Kohle durch Vergasen eines in einem Reaktor im Gegenstrom zu dem Dampf und Sauerstoff oder Luft enthaltenden Strom absteigenden Bettes aus mit Katalysator imprägnierter Kohle, wobei der Luftgehalt nicht mehr als 35 Gew.-

% der trockenen, aschefreien Kohle beträgt oder der Sauerstoffgehalt 1 bis 25 Gew.-% der trocknen aschefreien Kohle beträgt, Unter einem über 24,5165 bar (25 Atmosphären absolut) liegenden Druck und bei einer Temperatur von 400°C bis 850°C;

- Strippen des nicht reagierten Dampfes und der kondensierbaren Stoffe von dem solcherart gebildeten Gas, und
- Entsäuern des so gebildeten Gases.

2. Verfahren nach Anspruch 1, worin die Methanisierungsstufe bei einer Temperatur von 450°C bis 750°C ausgeführt wird.

3. Verfahren nach Anspruch 1, worin die Pelletgröße der mit Katalysator imprägnierten Kohle von 3 mm bis 2 cm beträgt.

4. Verfahren nach Anspruch 1, welches weiterhin eine ergänzende, bei einer Temperatur von 350°C bis 500°C ausgeführte Methanisierungsstufe umfaßt.

5. Verfahren nach Anspruch 1, worin die Methanisierungsstufe mit einer Dampfmenge von 100 bis 200 Gew.-%, bezogen auf die trockene, aschefreie Kohle, ausgeführt wird.

6. Verfahren nach Anspruch 1, worin die Verweilzeit der in Methan ueberzufuehrenden Kohle im Methanisierungsreaktor von 30 Minuten bis 30 Stunden beträgt.

7. Verfahren nach Anspruch 6, worin die Verweilzeit von einer Stunde bis 5 Stunden beträgt.

8. Verfahren nach Anspruch 1, worin die Methanisierungsstufe in einem Reaktor erfolgt, worin die Temperatur vom Reaktorkopf zum Reaktorboden zunimmt.